# EUROPEAN PATENT SPECIFICATION

(11) **EP 0 585 914 B1**
(45) Date of publication and mention of the grant of the patent: **16.07.1997**
(21) Application number: 93114025.5
(22) Date of filing: 02.09.1993
(51) Int. Cl.: G01N 33/558, G01N 33/546

(54) **Chromatographic sandwich test for specific antibody and device therefor**
Chromatographischer Sandwichtest für spezifischen Antikörper und Vorrichtung dafür
Test de sandwich chromatographique pour un anticorps spécifique et dispositif l'utilisant

(30) Priority: 04.09.1992 US 941350
(43) Date of publication of application: 09.03.1994
(73) Proprietor: Becton Dickinson and Company, Franklin Lakes, New Jersey 07417-1880 (US)
(72) Inventor: Lovell, Stephen J., Towson, Maryland 21204 (US)
(74) Representative: von Kreisler, Alek, Dipl.-Chem.

(56) References cited:
- EP-A- 0 196 880
- EP-A- 0 291 194
- EP-A- 0 462 376
- WO-A-91/12528
- US-A- 4 228 237

## Description

### BACKGROUND OF INVENTION

Immunoassays are now well known tools of the diagnostic industry. Such assays take advantage of the specificity of the antigen antibody reaction to detect the presence of the analyte in the test sample. This provides a simple method for measuring the analyte. See for example EP-A-0 291 194.

When the target analyte is an antibody, the assay ordinarily uses an anti-species antibody coated on the detector, such as anti-IgG or anti-IgM. This type of detector is difficult to incorporate into a single step assay, as it would tend to bind both specific and non-specific antibody, if the non-specific antibody is in excess in the sample. Such is generally the case with serum.

Thus, there exists a need for alternate technologies to utilize such a single step assay.

### SUMMARY OF INVENTION

This invention presents a simple and rapid single step assay for the detection of specific antibodies in a sample. Specifically, the test utilizes a two zone chromatographic device wherein the two zones are a detector zone and a capture zone.

The detector zone is comprised of detector particulates coated with an antigen or antigen analog and the capture zone is antigen, or antigen analog, bound to the chromatographic matrix. In the assay, the sample containing the specific antibody is applied to the device upstream of the detector zone, which, in turn, is upstream of the capture zone. The specific antibodies will first bind to the detector zone particulates, and these complexes will become bound to the capture zone, where the detector signal can be read.

### BRIEF DESCRIPTION OF THE FIGURES

FIGURE 1 depicts a chromatographic device of this invention showing the chromatographic matrix (1), detector zone (2), and capture zone.

FIGURE 2 depicts the complex formed in the capture zone showing the detector (1), antigen on the detector surface (2), antigen bound to the chromatographic matrix (3) and specific antibody (4).

### DETAILED DESCRIPTION OF INVENTION

The detector zone comprises particles of detector coated with antigen. For a true one step assay, the detector should provide a visible readout, although other detectors (e.g. fluorescent, UV) can also be used, the only criterion being the ability to attach antigens to its surface.

The detector is a particulate material. One useful particulate is a sac which includes a dye or other colored substance as a readout. The sac which is used may be any one of a wide variety of sacs, including but not limited to intact erythrocytes, erythrocyte ghosts, liposomes (single walled, sometimes called vesicles or multilamellar), polymer microcapsules (for example, those made by coascervation, or interfacial polymerization), etc.

Also useful are colored particles (e.g., latex or other polymeric particles) and precipitated or insoluble metals, nonmetals and alloys.

The primary criterion for selection of the detector are the ability to bind the antigen and the visibility of the readout under test conditions.

The signal-generating substance can be any substance which exhibits a detectable signal under test conditions, but is preferably a visibly colored dye or pigment. Other dyes, such as UV or fluorescent, can also be used, but will complicate detection by necessitating the use of specialized equipment. Most preferably the detector is a colored latex particle.

The antigen is attached to the surface of the detector, by means which are well known in the art, in a geometric orientation which renders it accessible to reaction with specific antibody in the sample to form a complex. This complex then migrates through the chromatographic matrix to the capture zone which contains antigen immobilized on the matrix by means which are well known in the art (in the case of low molecular weight antigen, it may be necessary to couple the antigen to a carrier macromolecule before immobilization on the chromatographic matrix). The multivalent specific antibody also binds to the bound antigen in this zone to form a labeled antigen/antibody/bound antigen "sandwich," which can then be detected.

The assay is highly selective for specific antibody, since it relies on the formation of an antigen/antibody complex which is similarly highly specific.

The chromatographic matrix can be any of the various matrices known in the art including silica, polyacrylamide alumina, and, preferably, nitrocellulose. Further, while the assay is preferably performed in a thin layer chromatograph device (TLC) format, it can also be performed in any other compatible chromatographic formats such as column chromatography.

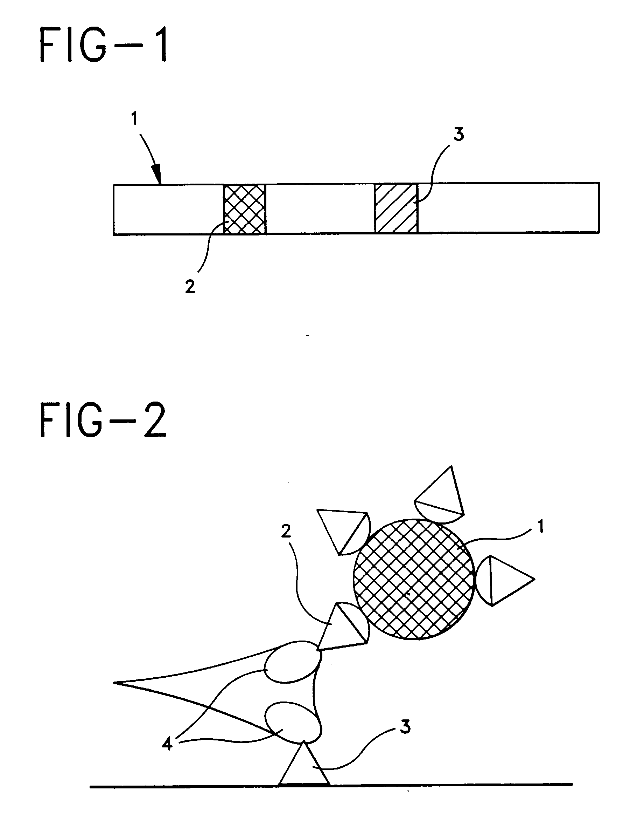

In the preferred embodiment, the TLC device is arranged as shown in FIG. 1. Briefly, the sample is introduced to the chromatographic medium (1) upstream of the detector zone (2). After migrating through the detector zone, the sample continues on to the capture zone (3) where any antibody/antigen complexes are bound and detected.

### EXAMPLES

The following examples illustrate certain preferred embodiments of this invention, but are not intended to be illustrative of all embodiments.

### EXAMPLE 1 - Anti-biotin system

In this example, a TLC device containing 8 um nitrocellulose as the chromatographic matrix, was arranged as in FIG. 1. The detector zone contained 3 ul of biotinylated red latex, and the capture zone contained biotinylated BSA sprayed onto the device as a straight line with a CAMAG TLC applicator. The density of biotinylated BSA on the membrane was lug/cm.

The sample was placed on the surface of the device upstream of the detector zone, and the system was visually observed for the development of a red color in the capture zone. Two samples were tested, normal rabbit serum, and rabbit serum containing anti-biotin antibody.

Only the serum containing the specific anti-biotin antibody gave a positive result.

A third sample containing normal rabbit serum seeded with 20 ug/ml of affinity purified anti-biotin antibody was similarly tested, and yielded a positive result indicating the sensitivity is at least 20 ug/ml. Triton™ X-100 was added (final conc. 1% (w/V) to all samples in this example to ensure rapid migration of detector latex.

### EXAMPLE 2 - Anti-rabbit IgG system

In this example, a device similar to that used in Example 1 was utilized, except that the detector was blue colored 0.48 um latex particles (0.48 um) coated with rabbit IgG and the capture zone was rabbit IgG spotted by micropipettor on the nitrocellulose (0.4 mg/ml IgG, 3 ul/spot); the blue latex detector was mixed 1:1 (V/V) with a solution consisting of PBS, 10% BSA, 5% sucrose, 0.2% Triton™ X-100, pH 7.7, prior to application to the nitrocellulose.

Three sera were tested, normal goat serum (diluted 1/20), goat serum containing anti-rabbit IgG (diluted 1/20), and goat serum containing anti-rabbit IgG (diluted 1/20) with normal rabbit serum added to 10% (V/V).

Only the second sample produced a positive result, indicating the specificity of the anti-rabbit antibody, since normal rabbit serum mired in the third sample (which contains rabbit IgG) will bind to the anti-rabbit IgG and block the reaction.

## Claims

1. A device for performing single step immunoassays for specific antibodies to an antigen comprising a chromatographic matrix containing a detector zone and a capture zone downstream of said detector zone, wherein the detector zone comprises detector particulates coated with the antigen or an antigen analog and the capture zone contains the antigen or antigen analog bound to the chromatographic matrix.

2. The device of Claim 1 wherein the chromatographic matrix is selected from the group consisting of silica, nylon, glass fiber, polyacrylamide, alumina, and nitrocellulose.

3. The device of Claim 1 wherein the detector particulates produce a visible readout.

4. The device of Claim 1 wherein the detector is selected from the group consisting of precipitated or insoluble metals, precipitated or insoluble nonmetals, sacs containing a dye or colored substance, and precipitated or insoluble alloys.

5. A method for determining the presence of specific antibody to an antigen in a sample comprising:
(i) applying the sample to a chromatographic matrix of a device, containing a detector zone and a capture zone, wherein sample is applied upstream of said detector zone;
(ii) allowing said sample to migrate firstly through said detector zone, which contains detector particulates coated with said antigen or an antigen analog, such that antigen-antibody complexes are formed, and secondly through said capture zone, which contains the antigen or an antigen analog bound to said chromatographic matrix, such that the antigen-antibody complexes formed will bind to said antigen or antigen analog; and
(iii) observing the capture zone for the presence of particulates, wherein the presence of particulates indicates the presence of the specific antibody.

6. The method of Claim 5 wherein the chromatographic matrix is selected from the group consisting of silica, nylon, glass fiber, polyacrylamide, alumina, and nitrocellulose.

7. The method of Claim 5 wherein the detector particulates produce a visible readout.

8. The method of Claim 5 wherein the detector particulate a sac containing a dye or colored substance.

9. The method of Claim 5, wherein the detector is selected from the group consisting of precipitated or insoluble metals, precipitated or insoluble nonmetals and precipitated or insoluble alloys.

10. The method of Claim 5 wherein the device is in a thin layer chromatographic format.

## Patentansprüche

1. Vorrichtung zur Durchführung von einstufigen Immunoassays für spezifische Antikörper gegen ein Antigen, umfassend eine chromatographische Matrix, die eine Detektorzone und eine Abfangzone stromabwärts von der Detektorzone enthält, wobei die Detektorzone Detektorteilchen umfaßt, die mit einem Antigen oder Antigenanalogon beschichtet sind, und die Abfangzone das Antigen oder Antigenanalogon enthält, das an die chromatographische Matrix gebunden ist.

2. Vorrichtung gemäß Anspruch 1, wobei die chromatographische Matrix aus der Gruppe ausgewählt ist, die aus Siliciumoxid, Nylon, Glasfaser, Polyacrylamid, Aluminiumoxid und Nitrocellulose besteht.

3. Vorrichtung gemäß Anspruch 1, wobei die Detektorteilchen eine sichtbare Ablesung ergeben.

4. Vorrichtung gemäß Anspruch 1, wobei der Detektor aus der Gruppe ausgewählt ist, die aus gefällten oder unlöslichen Metallen, gefällten oder unlöslichen Nichtmetallen, einen Farbstoff oder eine farbige Substanz enthaltenden Beuteln und gefällten oder unlöslichen Legierungen besteht.

5. Verfahren zur Bestimmung der Gegenwart eines spezifischen Antikörpers gegen ein Antigen in einer Probe, umfassend:
(i) Auftragen der Probe auf eine chromatographische Matrix einer Vorrichtung, die eine Detektorzone und eine Abfangzone enthält, wobei die Probe stromaufwärts von der Detektorzone aufgetragen wird;
(ii) Wandernlassen der Probe zuerst durch die Detektorzone, die Detektorteilchen enthält, die mit dem Antigen oder einem Antigenanalogon beschichtet sind, so daß sich Antigen-Antikörper-Komplexe bilden, und zweitens durch die Abfangzone, die an die chromatographische Matrix gebunden das Antigen oder ein Antigenanalogon enthält, so daß die gebildeten Antigen-Antikörper-Komplexe an das Antigen oder Antigenanalogon binden; und
(iii) Beobachten der Abfangzone im Hinblick auf die Gegenwart von Teilchen, wobei die Gegenwart von Teilchen die Gegenwart des spezifischen Antikörpers anzeigt.

6. Verfahren gemäß Anspruch 5, wobei die chromatographische Matrix aus der Gruppe ausgewählt ist, die aus Siliciumoxid, Nylon, Glasfaser, Polyacrylamid, Aluminiumoxid und Nitrocellulose besteht.

7. Verfahren gemäß Anspruch 5, wobei die Detektorteilchen eine sichtbare Ablesung ergeben.

8. Verfahren gemäß Anspruch 5, wobei das Detektorteilchen ein Beutel ist, der einen Farbstoff oder eine farbige Substanz enthält.

9. Verfahren gemäß Anspruch 5, wobei der Detektor aus der Gruppe ausgewählt ist, die aus gefällten oder unlöslichen Metallen, gefällten oder unlöslichen Nichtmetallen und gefällten oder unlöslichen Legierungen besteht.

10. Verfahren gemäß Anspruch 5, wobei die Vorrichtung in einem dünnschichtchromatographischen Format vorliegt.

## Revendications

1. Dispositif pour mettre en oeuvre des dosages immunologiques, en une seule étape, d'anticorps spécifiques dirigés contre un antigène, comprenant une matrice chromatographique contenant une zone de détection et une zone de capture en aval de ladite zone de détection, dans lequel la zone de détection comprend des matières particulaires détectrices recouvertes de l'antigène ou d'un analogue d'antigène et la zone de capture contient l'antigène, ou un analogue d'antigène, fixé à la matrice chromatographique.

2. Dispositif selon la revendication 1, dans lequel la matrice chromatographique est choisie dans le groupe constitué par la silice, le nylon, la fibre de verre, le polyacrylamide, l'alumine, et la nitrocellulose.

3. Dispositif selon la revendication 1, dans lequel les matières particulaires détectrices produisent un affichage visible.

4. Dispositif selon la revendication 1, dans lequel le détecteur est choisi dans le groupe constitué par les métaux précipités ou insolubles, les non-métaux précipités ou insolubles, les sacs contenant un colorant ou une substance colorée, et les alliages précipités ou insolubles.

5. Procédé pour déterminer la présence d'anticorps spécifique dirigé contre un antigène dans un échantillon, comprenant les étapes qui consistent à:
(i) déposer l'échantillon sur une matrice chromatographique d'un dispositif contenant une zone de détection et une zone de capture, l'échantillon étant déposé en amont de ladite zone de détection;
(ii) laisser migrer ledit échantillon tout d'abord à travers ladite zone de détection, qui contient des matières particulaires détectrices recouvertes dudit antigène ou d'un analogue d'antigène, de sorte qu'il se forme des complexes antigène-anticorps, et ensuite à travers ladite zone de capture, qui contient l'antigène, ou un analogue d'antigène, fixé à ladite matrice chromatographique, de sorte que les complexes antigène-anticorps formés se fixent audit antigène ou analogue d'antigène; et
(iii) observer la présence de matières particulaires dans la zone de capture, la présence de matières particulaires indiquant la présence de l'anticorps spécifique.

6. Procédé selon la revendication 5, dans lequel la matrice chromatographique est choisie dans le groupe constitué par la silice, le nylon, la fibre de verre, le polyacrylamide, l'alumine, et la nitrocellulose.

7. Procédé selon la revendication 5, dans lequel les matières particulaires détectrices produisent un affichage visible.

8. Procédé selon la revendication 5, dans lequel la matière particulaire détectrice est un sac contenant un colorant ou une substance colorée.

9. Procédé selon la revendication 5, dans lequel le détecteur est choisi dans le groupe constitué par les métaux précipités ou insolubles, les non-métaux précipités ou insolubles et les alliages précipités ou insolubles.

10. Procédé selon la revendication 5, dans lequel le dispositif est dans une présentation chromatographique en couche mince.
